(19) Europäisches Patentamt
European Patent Office
Office européen des brevets

(11) **EP 2 025 299 A1**

(12) **DEMANDE DE BREVET EUROPEEN**

(43) Date de publication:
**18.02.2009 Bulletin 2009/08**

(51) Int Cl.:
*A61B 18/20* (2006.01)

(21) Numéro de dépôt: **07370014.8**

(22) Date de dépôt: **16.08.2007**

(84) Etats contractants désignés:
**AT BE BG CH CY CZ DE DK EE ES FI FR GB GR HU IE IS IT LI LT LU LV MC MT NL PL PT RO SE SI SK TR**
Etats d'extension désignés:
**AL BA HR MK RS**

(71) Demandeur: **Optical System & Research for Industry and Science Osyris 59260 Hellemmes (FR)**

(72) Inventeurs:
- **Zemmouri, Jaouad**
  **59242 Genech (FR)**
- **Ringot, Jean**
  **59370 Mons-En-Baroeul (FR)**
- **Wassmer, Benjamin**
  **59139 Wattignies (FR)**

(74) Mandataire: **Matkowska, Franck**
**Matkowska & Associés**
**9 Rue Jacques Prévert**
**59650 Villeneuve d'Ascq (FR)**

(54) **Procédé et système de contrôle d'un traitement par irradiation sous-cutannee ou intra-cutannee au moyen d'un rayonnement electromagnétique**

(57)     Pour contrôler automatiquement un traitement au cours duquel on effectue une irradiation sous-cutanée ou intra-cutanée au moyen d'un rayonnement électromagnétique de traitement et éventuellement au moyen d'un rayonnement électromagnétique de visée, on met en oeuvre les étapes suivantes :

- acquisition de plusieurs images I(t) successives de la zone traitée au moyen d'un capteur (1) externe qui est sensible à la longueur d'onde ou dans la gamme de longueurs d'onde du rayonnement électromagnétique de traitement ou du rayonnement électromagnétique de visée , l'intervalle de temps ($\tau$) entre deux images successives [I(t-1) ; I(t)] étant connu,

- détection et localisation dans chaque image I(t) d'un point lumineux p(t) correspondant au spot d'irradiation (S) du rayonnement électromagnétique de traitement ou du rayonnement électromagnétique de visée,

- calcul pour chaque point lumineux p(t) de l'un au moins des paramètres suivants : l'énergie délivrée [$e_{ij}(t)$ ou $E_{ij}(t)$] à partir de la puissance P(t) du rayonnement électromagnétique de traitement et de l'intervalle de temps ($\tau$) entre deux images successives [I(t-1) ; I(t)] ; la vitesse de déplacement v(t) du spot d'irradiation (S) à partir des positions de deux points lumineux [p(t-1) ; p(t)] dans deux images différentes [I(t-1) ; I(t)] et de l'intervalle de temps entre ces deux images [I(t-1) ; I(t)].

FIG.5

**Description**

Domaine technique

La présente invention concerne le domaine du traitement du corps humain ou animal par irradiation sous-cutanée ou intra-cutanée au moyen d'un rayonnement électromagnétique. Dans ce domaine, elle concerne plus particulièrement une nouvelle solution technique de contrôle de l'énergie du rayonnement électromagnétique appliquée au cours d'un traitement, ladite solution trouvant son application dans différents types d'endotraitements thérapeutiques ou cosmétiques sous-cutanés ou intra-cutanés, tels par exemple la lipolyse, les traitements endoveineux, le remodelage ou la cicatrisation de la peau par chauffage du collagène présent dans le derme et/ou par stimulation thermique des fibroblastes pour accélérer la production de collagène dans le derme. L'invention a également pour objet un nouveau procédé de remodelage ou de cicatrisation de la peau.

Art antérieur

Dans le domaine des traitements thérapeutiques ou cosmétiques du corps humain ou animal, on utilise à ce jour différentes solutions techniques basées sur une irradiation sous-cutanée de la zone à traiter au moyen d'un rayonnement électromagnétique, et notamment sur une irradiation au moyen d'un rayonnement électromagnétique produit par exemple dans le domaine de longueurs d'onde du visible en utilisant un faisceau laser continu ou pulsé à différentes puissances. Dans ces traitements sous-cutanés, on introduit le rayonnement électromagnétique sous la peau jusqu'à la zone à traiter, au moyen par exemple d'une aiguille creuse ou d'une canule, dans laquelle est introduite une fibre optique reliée à une source de rayonnement électromagnétique adaptée, par exemple un laser.
Parmi les traitements par irradiation électromagnétique sous-cutanée, on peut citer principalement, mais de manière non exhaustive, la lipolyse, qui consiste à détruire, notamment par effets thermiques, les cellules adipeuses présentes dans l'hypoderme en introduisant dans l'hypoderme, à différentes profondeurs, l'extrémité distale de la fibre optique par laquelle sort le rayonnement électromagnétique. On peut également citer tous les traitements endoveineux dans lesquels le rayonnement électromagnétique est produit dans une veine. Pour la lipolyse laser, on pourra se référer par exemple aux publications suivantes : US 6 206 873, US 5 954 710, US 2006/0224148. Pour les traitements endoveineux par laser, on pourra se référer par exemple aux publications US 4 564 011, US 5 531 739, US 6 398 777.
Une difficulté majeure de ces traitements est liée aux risques de destruction irréversible par effet thermique de cellules non ciblées dans la zone traitée, voire même dans une zone contiguë à la zone traitée. Ce risque dépend non seulement de la puissance et de la longueur d'onde du rayonnement électromagnétique, mais également et surtout de la vitesse à laquelle le spot d'irradiation électromagnétique est déplacé dans la zone à traiter. Or ce dernier paramètre de vitesse de déplacement dépend le plus souvent d'une action manuelle humaine mise en oeuvre par le praticien réalisant le traitement, et est donc une source importante de risques.
Pour tenter de résoudre cette difficulté, on a déjà cherché à ce jour à contrôler l'énergie du rayonnement électromagnétique appliqué lors du traitement. Par exemple, dans la demande de brevet US 2004/0199151, on propose une solution basée sur une mesure de la vitesse de retrait de la fibre optique, et sur un contrôle automatique de la puissance du laser, en fonction de la vitesse mesurée, en sorte de maintenir une énergie de traitement constante appropriée. Différentes solutions de mesure de vitesse de déplacement de la fibre optique sont envisagées. Par exemple, on détecte automatiquement des marques spécifiques faites sur une certaine longueur de la fibre optique, ou on met en oeuvre un dispositif optique de mesure de vitesse dans lequel passe la fibre optique. Cette solution présente deux inconvénients. D'une part, les moyens de mesure de la vitesse de déplacement de la fibre optique sont positionnés dans le champ opératoire, ce qui pose un problème de stérilité de ces moyens de mesure. D'autre part, cette solution ne permet pas une localisation de la zone effectivement traitée, et en particulier ne permet pas de cartographier les doses d'énergie appliquées en chaque point de la zone effectivement traitée.
D'autres solutions de contrôle basées sur une détection extérieure de la température de la peau au moyen par exemple d'un détecteur infrarouge ou de réactifs thermosensibles appliqués sur la peau ont également été proposées. Cependant, ces solutions ne sont pas satisfaisantes, à cause notamment du temps de propagation de la chaleur jusqu'à la surface de la peau. Lorsque le seuil de température de la peau est atteint et détecté, il est généralement trop tard, et des lésions thermiques irréversibles sous-cutanées ont pu déjà être causées.
Dans la demande de brevet internationale WO 2006/107522, il est proposé une solution de lipolyse par laser, dans laquelle le faisceau laser est introduit dans l'hypoderme au moyen d'un ensemble canule/fibre optique. Un objectif dans cette publication est de protéger le derme contre les effets thermiques destructifs du faisceau laser en s'assurant que l'extrémité distale de la fibre optique, lors d'un tir, n'est pas située dans le derme, mais est située dans l'hypoderme en étant suffisamment éloignée du derme. Dans ce but, on contrôle la profondeur du tir laser, en détectant au moyen d'un capteur optique extérieur l'intensité de l'énergie lumineuse du tir, qui traverse les différentes couches (hypoderme, derme, épiderme), et qui est visible par le capteur depuis l'extérieur. Plus l'intensité est élevée, et plus la profondeur du tir laser est faible. Cette solution ne permet pas toutefois de contrôler l'énergie appliquée lors du traitement et en particulier ne permet pas de cartographier les doses d'énergie appliquées en chaque point de la zone effectivement traitée.

En dehors des traitements sous-cutanés précités, il existe également, dans le domaine de la dermatologie, des traitements thermiques de la peau de type non invasifs.

En particulier, on met en oeuvre des traitements thermiques non invasifs pour chauffer le collagène présent dans le derme de la peau et/ou stimuler thermiquement les cellules (fibroblastes) produisant le collagène afin de stimuler la production de collagène dans le derme par les fibroblastes.

Une application importante de ces traitements thermiques non invasifs du derme est le remodelage de la peau par le collagène afin de diminuer ou faire disparaître les rides dues au vieillissement, ou de supprimer les aspects inesthétiques de la peau dits « peau d'orange ».

Par exemple dans le brevet américain US 6 659 999 et dans la demande de brevet américain US 2003/0040739, on propose des solutions de remodelage de la peau par le collagène basées sur une irradiation électromagnétique externe de la peau, au moyen par exemple d'un exolaser.

La stimulation de la production du collagène par un laser externe peut également être utilisée pour obtenir une meilleure cicatrisation de la peau. Par exemple, une méthode de cicatrisation de la peau au moyen d'un laser à 815nm est décrite dans l'article : « Laser Assisted Skin Closure (LASC) by using a 815- nm Diode-Laser System Accelerates and Improves Wound healing », A. Capon et al, Lasers in Surgery and Medicine 28 :168-175 (2001).

Lors de ces traitements thermiques du derme, il est important d'obtenir un chauffage suffisant du derme, sans toutefois atteindre une température de coagulation (de l'ordre de 60°C) qui détruirait les fibroblastes.

Un inconvénient de ces traitements non invasifs réside dans les risques de brûlure de l'épiderme. En pratique, pour éviter ces risques de brûlure, on est contraint de combiner à ce traitement thermique, un refroidissement externe et local de l'épiderme.

Résumé de l'invention

Selon un premier aspect, l'invention vise à proposer une nouvelle solution technique pour le contrôle automatique et en temps réel d'un traitement par irradiation sous-cutanée ou intra-cutanée au moyen d'un rayonnement électromagnétique, dans le but de contrôler les doses d'énergie délivrées dans la zone traitée, et éviter notamment de délivrer des doses d'énergie excessives ou à l'inverse de délivrer des doses d'énergie trop faibles et inefficaces pour le traitement

L'invention a ainsi pour premier objet un procédé de contrôle automatique d'un traitement au cours duquel on effectue une irradiation sous-cutanée ou intra-cutanée au moyen d'un rayonnement électromagnétique de traitement et éventuellement au moyen d'un rayonnement électromagnétique de visée. Ce procédé de contrôle comprend les étapes suivantes :

- acquisition de plusieurs images I(t) successives de la zone traitée au moyen d'un capteur (1) externe qui est sensible à la longueur d'onde ou dans la gamme de longueurs d'onde du rayonnement électromagnétique de traitement ou du rayonnement électromagnétique de visée, l'intervalle de temps ($\tau$) entre deux images successives [I(t-1) ; I(t)] étant connu,
- détection et localisation dans chaque image I(t) d'un point lumineux p(t) correspondant au spot d'irradiation (S) du rayonnement électromagnétique de traitement ou du rayonnement électromagnétique de visée,
- calcul pour chaque point lumineux p(t) de l'un au moins des paramètres suivants : l'énergie délivrée [$e_{ij}(t)$ ou $E_{ij}(t)$ ] à partir de la puissance P(t) du rayonnement électromagnétique de traitement et de l'intervalle de temps ($\tau$) entre deux images successives [I(t-1) ; I(t)] ; la vitesse de déplacement v(t) du spot d'irradiation (S) à partir des positions de deux points lumineux [p(t-1) ; p(t)] dans deux images différentes [I(t-1) ; I(t)] et de l'intervalle de temps entre ces deux images [I(t-1) ; I(t)].

Pour la mise en oeuvre de l'invention, le spot d'irradiation détecté par le capteur peut selon le cas être le spot d'irradiation du rayonnement électromagnétique de traitement ou le spot d'irradiation du rayonnement électromagnétique de visée. Néanmoins, le rayonnement électromagnétique détecté au moyen du capteur sera de préférence le rayonnement électromagnétique de traitement, car celui-ci est en pratique plus puissant que le rayonnement électromagnétique de visée.

L'invention a également pour objet un système de contrôle d'un traitement par irradiation sous-cutanée ou intra-cutanée au moyen d'un rayonnement électromagnétique, tel que défini dans la revendication 10, et un appareil médical tel que défini dans la revendication 18, et incluant ledit système de contrôle.

Selon un deuxième aspect, l'invention a pour objectif de proposer un nouveau procédé de remodelage de la peau ou de cicatrisation de la peau par irradiation au moyen d'un rayonnement électromagnétique.

De manière caractéristique selon l'invention, l'irradiation au moyen du rayonnement électromagnétique est réalisée dans le plan sous-dermique. Cette irradiation par rayonnement électromagnétique dans le plan sous-dermique permet de chauffer le collagène présent dans le derme et/ou de stimuler thermiquement la production de collagène par un chauffage des fibroblastes.

Jusqu'à ce jour les solutions de traitement thermique permettant d'obtenir un remodelage ou une cicatrisation de la peau étaient le plus souvent non invasifs. On a certes également proposé des solutions invasives, notamment dans le brevet US 5 370 642, mais dans ce cas on veillait systématiquement jusqu'à ce jour à ce que la fibre optique du laser endoculaire (endolaser) pénètre dans l'hypoderme, à une profondeur suffisante pour que l'énergie soit délivrée à une distance suffisante du derme et éviter tout risque de dommage thermique pour le derme et l'épiderme.

C'est le mérite de l'invention d'avoir au contraire cherché à se rapprocher du derme et d'avoir montré qu'il était possible de réaliser un traitement thermique par irradiation au moyen d'un rayonnement électromagnétique délivré dans le plan sous-dermique, qui soit efficace pour obtenir un remodelage ou une cicatrisation de la peau par le collagène dans le derme, sans occasionner de dommage thermique irréversible pour le derme et l'épiderme.

Brève description des dessins

D'autres caractéristiques de l'invention apparaîtront plus clairement à la lecture de la description détaillée ci-après de plusieurs variantes de réalisation de l'invention données à titre d'exemples non limitatifs et non exhaustifs, laquelle description est faite en référence aux dessins annexés sur lesquels :

- la figure 1 est une vue en coupe d'une partie de corps humain montrant l'épiderme, le derme et l'hypoderme,
- la figure 2 représente de manière schématique un système de contrôle de l'invention,
- la figure 3 est un schéma bloc d'un appareil médical de l'invention,
- la figure 4 est une vue en coupe montrant un spot d'irradiation délivré dans le plan sous-dermique, ainsi que la camera d'un système de contrôle de l'invention qui est utilisée pour détecter et localiser ce spot d'irradiation,
- la figure 5 est un algorithme de cartographie des doses d'énergie délivrées pour un traitement endoveineux,
- les figures 5a, 5b et 5c représentent l'image qui est affichée sur un écran pour l'opérateur à différents stades du procédé de traitement, lors de la mise en oeuvre des étapes de l'organigramme de la figure 5,
- la figure 6 est un algorithme de cartographie des doses d'énergie délivrées pour un traitement de type lipolyse,
- la figure 7 est un algorithme de contrôle de la vitesse de déplacement du spot d'irradiation.

Description détaillée

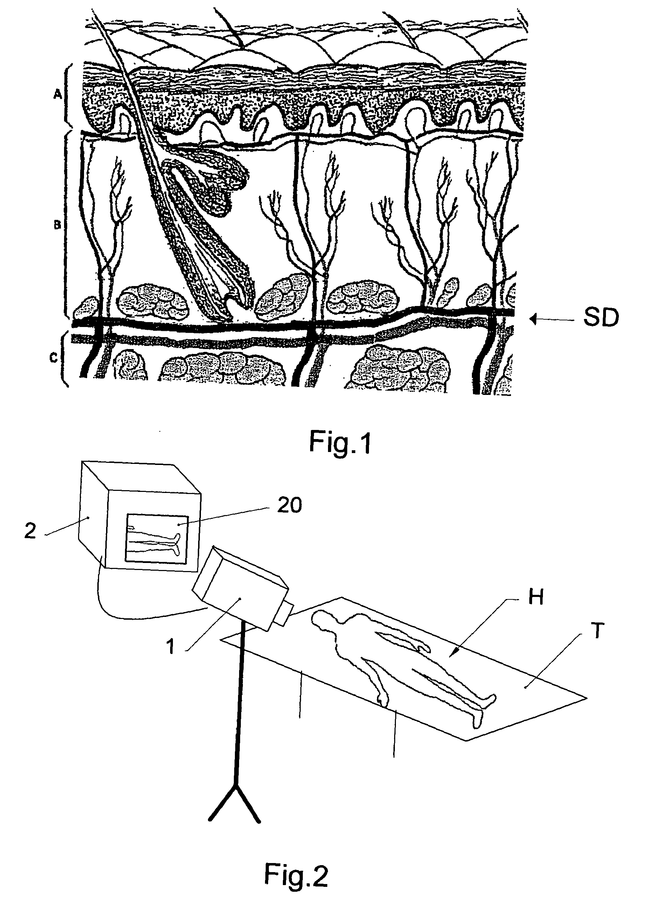

En référence à la figure 1, la peau est formée de deux couches principales : une couche superficielle mince A, composée de tissu épithélial et couramment appelée « épiderme » ; une couche B profonde et plus épaisse couramment appelée « derme ». En dessous de la peau, le fascia superficiel C, encore appelé « hypoderme » rattache le derme aux organes et tissus sous-jacents. Le plan SD situé immédiatement sous le derme, à l'interface entre le derme B et l'hypoderme C est communément désigné plan sous-dermique.

De manière usuelle, pour réaliser un traitement sous-cutané par laser, par exemple pour détruire les cellules adipeuses présentes dans l'hypoderme (lipolyse), on introduit une fibre optique dans l'hypoderme C, au moyen d'une canule ou aiguille creuse, après avoir le cas échéant pratiqué une petite incision dans l'épiderme A et dans le derme B. Cette fibre optique est reliée à son extrémité proximale (opposée à l'extrémité distale de la fibre introduite sous la peau) à une source laser pulsée ou continue. Cette source laser peut également être remplacée par tout autre type de source permettant de délivrer un rayonnement électromagnétique de traitement approprié, et par exemple par une source composée d'une ou plusieurs diodes de forte puissance. Une fois la fibre optique introduite, pour effectuer le traitement, le praticien déplace la fibre optique en effectuant des tirs laser. Deux techniques principales sont mises en oeuvre. Une première technique consiste à effectuer un retrait de la fibre de manière discontinue, et à effectuer un tir laser (continu ou pulsé) plus ou moins long à chaque arrêt. Une deuxième technique plus usitée car plus rapide, consiste à effectuer un retrait continu à vitesse sensiblement constante de la fibre et au cours de ce retrait continu à effectuer un tir laser (continu ou pulsé) sans interruption. En pratique, pour balayer toute la région à traiter, le praticien est amené à enfoncer plusieurs fois la fibre optique, en sorte de positionner initialement l'extrémité distale de la fibre optique dans différents points de la zone à traiter, et d'effectuer plusieurs opérations de retrait de la fibre optique avec tir laser (sans nécessairement sortir l'extrémité distale de la fibre optique).

De manière similaire, pour réaliser un traitement endoveineux par laser ou équivalent, il est usuel d'introduire une fibre optique dans une veine au moyen d'une canule ou aiguille creuse, et de traiter l'intérieur de la veine en effectuant des tirs lasers selon l'une ou l'autre des deux techniques discontinue ou continue précitées.

Le système et le procédé de contrôle de l'invention, dont un exemple de réalisation va à présent être détaillé, permet avantageusement de contrôler, dans ce type d'endotraitements par irradiation au moyen d'un rayonnement électromagnétique, l'énergie du rayonnement électromagnétique délivrée dans la zone traitée.

Il convient de souligner que jusqu'à ce jour, dans le cas des traitements sous-cutanés par laser, afin d'éviter les risques de brûlure du derme B, et de l'épiderme C, on veille systématiquement à introduire l'extrémité distale de la fibre optique dans l'hypoderme à une profondeur suffisante dans l'hypoderme pour que le tir laser ou équivalent ne soit pas réalisé à proximité immédiate du derme B. Grâce au contrôle de l'énergie du rayonnement électromagnétique de traitement obtenu au moyen de l'invention, il devient possible de réaliser de nouveaux traitements sous-cutanés à proximité du derme ou dans le plan dit « sous dermique », à l'interface entre le derme et l'hypoderme, voire de réaliser des traitements intra-cutanés, en positionnant l'extrémité distale de la fibre optique dans le derme.

On a représenté de manière schématique sur la figure 2, un patient H allongé sur une table d'opération T. Sur cette figure, les moyens permettant un endotraitement par irradiation au moyen d'un rayonnement électromagnétique (laser de traitement, laser de visée, fibre optique et canule pour l'insertion et le guidage de la fibre optique) ne sont pas représentés.

Le système de contrôle de l'invention comporte une caméra 1, permettant d'acquérir successivement une pluralité d'images I(t) de la zone traitée, avec une vitesse de capture prédéfinie (intervalle de temps entre deux images I(t)).

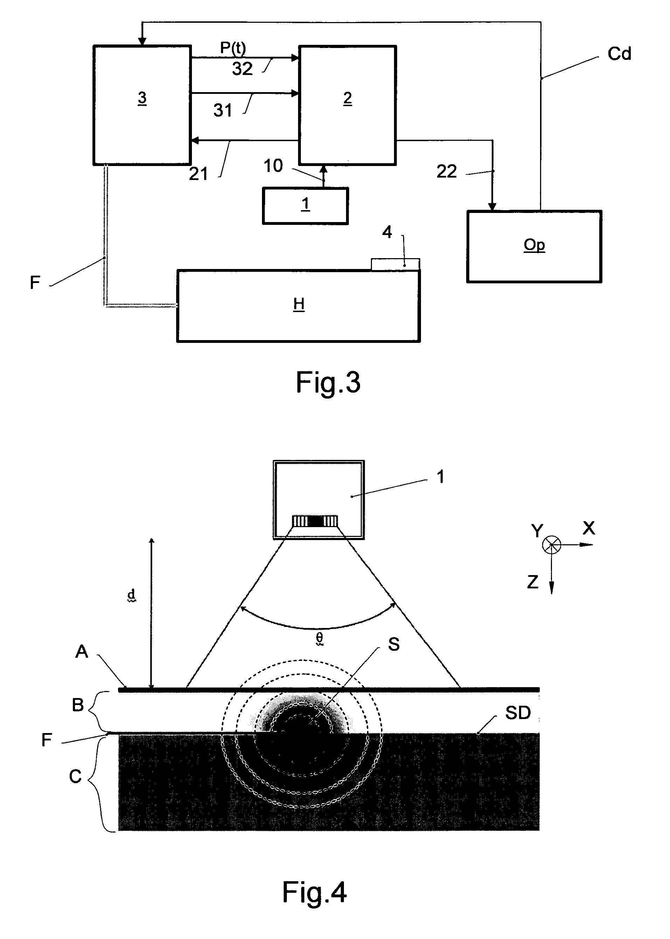

Sur la figure 3, on a représenté une source 3 permettant de délivrer un rayonnement électromagnétique de traitement, et reliée à la fibre optique F dont l'extrémité distale est introduite dans le corps du patient H. Cette source 3 est par exemple un laser. L'opérateur Op (praticien) pratiquant le traitement peut de manière usuelle commander le déclenchement et l'arrêt manuel d'un tir au moyen d'une commande Cd.

A chaque tir, le rayonnement électromagnétique produit peut être de type pulsé ou de type continu.

Le cas échéant, l'opérateur Op peut également régler la puissance P(t) de la source 3. Dans le présent texte, on désigne par « puissance P(t) de la source 3» la puissance moyenne de la source. Dans le cas d'un rayonnement électromagnétique continu, la puissance P(t) est égale à la puissance instantanée du rayonnement électromagnétique délivré par la source 3. Dans le cas d'un rayonnement électromagnétique de type pulsé, la puissance moyenne P(t) est égale à l'énergie délivrée sur une seconde. Par exemple, pour un laser de puissance instantanée ($P_{crête}$) de 1000 W et ayant une largeur d'impulsion (T) de 150 µs avec un taux de répétition (f) de 40 Hz :

- l'énergie E est égale à 6J
  ( $E = P_{crête} \times T \times f$ ) et

  - la puissance moyenne P(t) est égale à 6W ( $P(t) = E / 1s$ ).

Egalement, de manière connue en soi, l'appareil médical de traitement peut comporter une deuxième source de rayonnement électromagnétique (par exemple une deuxième source laser), qui est également reliée à la fibre optique F, et qui permet de délivrer un deuxième rayonnement électromagnétique dit de visée. Ce rayonnement électromagnétique de visée est généralement de plus faible puissance que le rayonnement électromagnétique de traitement délivré par la source 3. Ce rayonnement électromagnétique de visée est utilisé pour localiser la zone sous-cutanée ou intra-cutanée qui doit être traitée par le rayonnement électromagnétique de traitement.

En référence aux figures 2 et 3, la caméra 1 est reliée à des moyens électroniques de traitement 2, pour le traitement automatique du signal 10 délivré par la caméra. De préférence ces moyens électroniques de traitement 2 comportent des moyens de visualisation 20 (écran 20/ figure 1) permettant d'afficher pour l'opérateur Op (praticien) l'image de la zone traitée, et également, tel que cela apparaîtra plus clairement ultérieurement, de superposer à cette image une cartographie des doses d'énergie délivrées dans la zone traitée.

En référence à la figure 3, les moyens électroniques de traitement 2 communiquent également avec la source 3. Plus particulièrement, les moyens électroniques de traitement 2 sont informés par la source 3, au moyen du signal 31, si un tir est en cours ou non (source 3 activée ou non). Les moyens électroniques de traitement 2 sont également informés par la source 3, au moyen du signal 32, de la puissance moyenne P(t) du tir, laquelle puissance peut le cas échéant être réglable par l'opérateur Op.

Dans l'exemple particulier de la figure 3, les moyens électroniques de traitement 2 sont également conçus pour commander l'arrêt de la source 3 au moyen d'un signal de commande 21, et pour délivrer un signal d'avertissement 22 (sonore et/ou visuel) à destination de l'opérateur Op, par exemple lorsque l'énergie délivrée en un point atteint un seuil d'énergie prédéfini.

On a représenté de manière schématique sur la figure 4, une coupe transversale montrant une fibre optique F introduite dans le plan sous-dermique (entre le derme B et l'hypoderme C), au cours d'un tir.

La caméra 1 est positionnée à une distance (d) de la zone traitée et présente un angle de vision θ prédéfini. De préférence, mais non nécessairement, au cours du traitement la caméra 1 est fixe, la distance (d) et l'angle de vision θ étant réglés de telle sorte que la champ de vision de la camera 1 couvre toute la surface de la zone à traiter. Néanmoins, dans une autre variante, la camera 1 pourrait être mobile en sorte de couvrir toute la zone à traiter.

La caméra 1 est en outre choisie en sorte d'être sensible à la longueur d'onde ou dans la gamme de longueurs d'onde du rayonnement électromagnétique que l'on cherche à détecter.

De préférence, le rayonnement électromagnétique détecté est le rayonnement électromagnétique de traitement délivré par la source 3. Néanmoins, pour la mise en oeuvre de l'invention, le rayonnement électromagnétique détecté peut également être le rayonnement électromagnétique de visée.

Dans la suite de la description, le contrôle du traitement par irradiation est basé sur une détection par la caméra 1 du spot d'irradiation (S) du rayonnement électromagnétique de traitement délivré par la source 3. Dans une autre variante de réalisation, le contrôle du traitement par irradiation peut être basé sur une détection par la caméra 1 du spot d'irradiation (S) du rayonnement électromagnétique de visée.

Dans la plupart des cas, le traitement est mis en oeuvre avec une source laser ou équivalent ayant une longueur d'onde d'émission dans la gamme du visible, généralement entre 600nm et 1400nm pour les lasers les plus usités. Par conséquent, la plupart du temps, on choisira la caméra 1 de telle sorte qu'elle puisse détecter un rayonnement électromagnétique dans le domaine du visible ou proche infra rouge. La caméra est par exemple une caméra matricielle à capteurs à transfert de charges, communément appelée caméra CCD (à base par exemple de cellules photosensibles en silicium dans le domaine du visible ou de germanium pour le proche infra rouge).

L'invention n'est toutefois pas limitée à cette plage de longueurs d'onde 600nm-1400nm, mais peut être mise en oeuvre avec des rayonnements en dehors de cette plage, par exemple avec un rayonnement HF (Hyperfréquence) ou RF (radiofréquence) . Dans ce cas, le capteur sera adapté à la longueur d'onde du rayonnement (par exemple capteur matriciel à base de diodes Schottky).

A titre d'exemple non limitatif, le procédé de contrôle de l'invention peut être mis en oeuvre avec une caméra CMOS 500x582 pixels permettant d'acquérir une image toute les 25 secondes, un angle de vision de $\theta=70°$ et une distance (d) entre la caméra et l'épiderme A de 20 cm. La surface visible sur la caméra est dans ce cas de 784 cm$^2$ (28cmx28 cm). Chaque pixel correspond donc à une surface de 0,27 mm$^2$ (0,52mmx0,52 mm).

Au cours d'un tir, à l'extrémité distale de la fibre optique F, le faisceau laser en sortant de la fibre optique forme un spot d'irradiation S (figure 4) dont l'énergie est maximale au centre (à proximité immédiate de la sortie de la fibre optique F), et dont l'énergie décroît en s'éloignant de la sortie de la fibre optique F. Le rayonnement électromagnétique de ce spot d'irradiation S traverse les différentes couches de la peau (derme B et épiderme A), et est détecté par la camera 1. La caméra 1 permet ainsi de détecter à travers la peau et de suivre dans le temps la position du spot d'irradiation S dans un plan (X,Y) sensiblement parallèle à la surface de l'épiderme A (figure 4), et sensiblement perpendiculaire à la direction Z (direction correspondant à la profondeur).

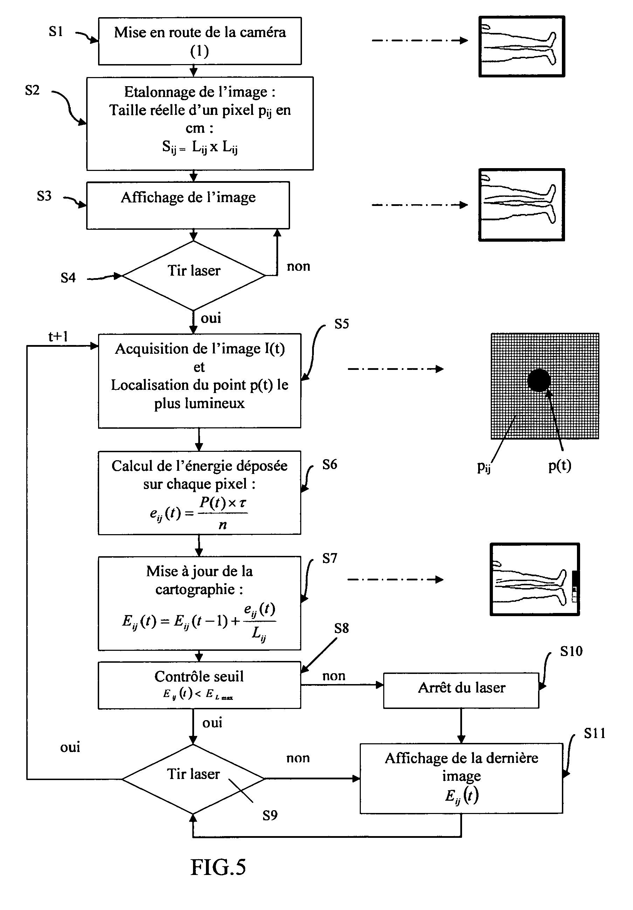

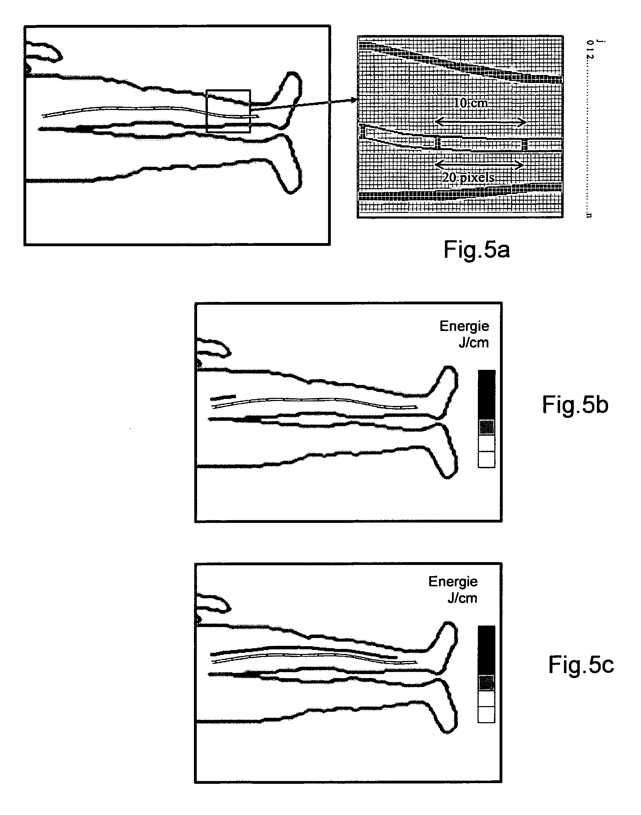

Les moyens électroniques de traitement 2 comportent une unité de traitement qui est programmée pour exécuter un algorithme, dont plusieurs variantes de réalisation sont représentées par les organigrammes respectivement des figures 5 à 7. Ces organigrammes des figures 5 à 7 sont donnés à titre d'exemples non limitatifs et non exhaustifs de l'invention.

Exemple d'algorithme de cartographie pour un traitement endoveineux - Figure 5

Cet algorithme permet de cartographier les doses d'énergie linéaire délivrées au cours d'un traitement endoveineux au cours duquel la fibre optique F est introduite à l'intérieur d'une veine et est retirée de la veine, selon un mouvement continu au cours duquel un tir est effectué sans interruption par l'opérateur (Op), ou selon un mouvement discontinu au cours duquel une pluralité de tirs successifs sont effectués par l'opérateur (Op) à différentes positions de l'extrémité distale de la fibre optique F.

Les étapes S1 à S3 sont des étapes de calibrage de la caméra 1, préalables à la réalisation du traitement endoveineux.

Etape S1 :

Au cours de cette première étape, l'opérateur Op met en route la caméra 1.

Etape S2 :

Ensuite, l'opérateur Op positionne sur la zone à visualiser un repère d'étalonnage 4 (par exemple règle graduée avec des graduations espacées d'une distance d1 connue). Les moyens électroniques de traitement 2 acquièrent au moyen de la caméra 1 une image réelle de la zone à traiter (figure 5a/ jambes du patient H avec le repère 4) et effectuent un étalonnage de l'image en calculant automatiquement, et de manière connue en soi, à partir des distances (d1) connues entre les graduations de la règle 4, la résolution ($S_{ij} = L_{ij} \times L_{ij}$) de chaque pixel, c'est-à-dire la surface réelle visualisée correspondant à un pixel de l'image. Dans l'exemple particulier de la figure 5a, la distance d1 entre deux graduations du repère 4 est de 10cm et couvre 20 pixels, ce qui correspond à une résolution ($S_{ij}$) de 5mmx5mm ( $L_{ij}$ = 5mm).

Etape S3 :

L'image réelle de la zone à traiter (incluant le repère d'étalonnage 4) est affichée sur l'écran 20 pour l'opérateur. Cette image correspond à la figure 5a.
Les étapes précitées de calibrage automatique permettent une mise en oeuvre du procédé quels que soient la distance (d) précitée entre la caméra 1 et l'épiderme A et le réglage la distance focale de la caméra

1. Lorsque ces paramètres sont constants d'un traitement à l'autre, il suffit de réaliser une seule fois le calibrage de la caméra, et il n'est pas nécessaire de reproduire les étapes de calibrage avant chaque traitement. Il convient en outre de souligner que ce calibrage de la caméra 1 est facultatif pour la mise en oeuvre de l'invention et se justifie uniquement pour les variantes particulières de réalisation dans lesquelles on cacule un paramètre fonction de la largeur $L_{ij}$ ou surface $S_{ij}$ d'un pixel.

Les étapes S4 à S10 mises en oeuvre au cours du traitement vont à présent être détaillées.

Etape S4 :

A partir du signal 31 précité délivré par la source 3, les moyens électroniques de traitement 2 détectent si un tir est en cours ou non. Si un tir est en cours, les moyens électroniques de traitement 2 exécutent automatiquement les étapes suivantes S5, S6,...

Etape S5 :

Les moyens électroniques de traitement 2 déclenchent l'acquisition d'une image I(t) au moyen de la caméra 1, et effectuent un filtrage de cet image I(t) pour détecter dans l'image le ou les pixels les plus lumineux formant le point p(t) le plus lumineux correspondant au spot réel d'irradiation S. D'une manière générale, ce point p(t) le plus lumineux forme une tache lumineuse qui selon le cas peut couvrir plusieurs pixels $p_{ij}$ de l'image (cas le plus fréquent et correspondant à l'exemple des figures annexées) ou ne couvrir qu'un seul pixel $p_{ij}$ de l'image. Ce point lumineux p(t) n'est pas nécessairement circulaire, mais en fonction du filtrage mis en oeuvre la tâche lumineuse détectée correspondant à ce point lumineux p(t) peut avoir une forme non circulaire.
Par exemple, pour le filtrage on met en oeuvre un simple seuillage de niveau de luminosité (niveau de gris dans le cadre d'une image monochrome) de type tout ou rien, en conservant uniquement les pixels dont le niveau de luminosité est supérieur à un seuil prédéfini. La dimension du point lumineux p(t) dépendra ainsi du niveau choisi pour le seuil de filtrage. Plus le seuil de filtrage est élevé, plus la dimension (en nombre de pixels) du point lumineux p(t) sera faible.
Le seuil de filtrage peut être fixe et prédéfini. Il peut également être paramétrable manuellement par l'opérateur afin notamment de tenir compte de la profondeur du traitement. Dans une variante de réalisation ce seuil de filtrage peut être auto adaptatif et calculé automatiquement à partir des niveaux de luminosité des pixels de l'image.

Etape S6 :

Les moyens électroniques de traitement 2 calculent automatiquement l'énergie $e_{ij}(t)$ du rayonnement électroma-gnétique, pour chaque pixel $p_{ij}$ du point lumineux p(t) qui a été détectée à l'étape précédente au moyen de la formule suivante :

$$(1) \quad e_{ij}(t) = \frac{P(t) \times \tau}{n}$$

dans laquelle :

- P(t) est la puissance moyenne du tir laser ; cette information est fournie aux moyens électroniques de traitement 2 par la source laser 3 (figure 4/ signal 32) ;
- $\tau$ est l'intervalle de temps séparant deux acquisitions d'image successives ; ce paramètre est caractéristique de la caméra 1 utilisée et dépend de la vitesse d'acquisition de la caméra 1 ; par exemple, si la vitesse d'acquisition de la caméra est de 25 images par seconde, $\tau$ vaut 40ms).
- n est le nombre de pixels couverts par le point lumineux p(t).

Dans cette variante de réalisation, l'énergie $e_{ij}(t)$ calculée pour chaque pixel $p_{ij}$ couvert par le point lumineux p(t) est identique.

Dans une autre variante de réalisation, on peut également effectuer un calcul d'énergie $e_{ij}(t)$ pour chaque pixel $p_{ij}$ couvert par le point lumineux p(t) qui est pondéré par l'intensité lumineuse de ce pixel $p_{ij}$. Etape S7 :

Les moyens électroniques de traitement 2 calculent pour chaque pixel $p_{i,j}$ du point lumineux détecté p(t), la nouvelle valeur d'énergie linéaire $E_{i,j}(t)$ (énergie par unité de longueur) au moyen de la formule suivante :

$$(2) \quad E_{ij}(t) = E_{ij}(t-1) + \frac{e_{ij}(t)}{L_{ij}}$$

dans laquelle :

- $E_{ij}(t-1)$ est la valeur d'énergie linéaire du pixel $p_{i,j}$ avant l'étape S6, étant précisé qu'au cours de la première itération $E_{ij}(t = 0)$ est nulle ;
- $e_{ij}(t)$ est la valeur calculée à l'étape précédente S6 ;
- $L_{ij}$ est la largeur d'un pixel calculée à l'étape S2 de calibrage.

Au cours de cette étape S7, les moyens électroniques de traitement 2 mettent à jour dans l'image affichée sur l'écran 20, l'intensité lumineuse $I_{ij}$ de chaque pixels $p_{i,j}$ correspondant au point lumineux p(t) détecté, à partir de la nouvelle valeur $E_{ij}(t)$calculées précédemment, cette intensité lumineuse $I_{ij}$ étant proportionnelle à $E_{ij}(t)$ . Par exemple, dans la cas d'une image monochrome, cette l'intensité lumineuse luminosité $I_{ij}$ est codée en niveau de gris à partir de la valeur d'énergie linéaire $E_{ij}$ qui a été calculée.

On réalise ainsi pour l'opérateur Op une cartographie de l'énergie électromagnétique pour chaque spot d'irradiation (S) détecté, en visualisant sur l'image réelle de la zone traitée acquise par la caméra 1, la position de chaque point lumineux détecté p(t) correspondant à un spot d'irradiation (S), et l'énergie linéaire délivrée $E_{ij}(t)$. Etape S8 :

Les moyens électroniques de traitement 2 vérifient si l'énergie linéaire $E_{i,j}(t)$ est acceptable, en comparant cette valeur avec un seuil prédéfini ($E_{L\ max}$), qui peut le cas échéant être réglable par l'opérateur Op.

Si l'énergie délivrée par unité de surface $E_{ij}(t)$ est supérieure à ce seuil, les moyens électroniques de traitement 2 commandent l'arrêt de la source 3 au moyen du signal 21 ( figure 4) et éventuellement déclenchent un signal 22 d'avertissement pour l'opérateur. Ce seuil est déterminé au cas par cas afin d'arrêter automatiquement la source 3, avant qu'un dommage thermique irréversible du derme et de l'épiderme ne se produise. Dans ce cas, le traitement est interrompu et les moyens électroniques de traitement 2 affichent pour l'opérateur Op la dernière image mise à jour (étape S11)

Si l'énergie linéaire $E_{ij}(t)$ est inférieure à ce seuil, les moyens électroniques de traitement 2 exécutent l'étape S9.

Dans une variante, plusieurs seuils d'alarme peuvent être prévus. Dans ce cas, le seuil précité ($E_{L\ max}$) correspondant au seuil le plus élevé. En cas de détection du dépassement d'un seuil intermédiaire plus faible (sans dépassement du seuil le plus élevé $E_{Lmax}$), les moyens électroniques de traitement 2 n'arrêtent pas la source 3, mais déclenchent un signal 22 d'avertissement (visuel et/ou sonore) pour l'opérateur Op, afin que celui-ci puisse réagir en temps réel, par exemple en accélérant la vitesse de retrait de la fibre optique F pour diminuer la dose d'énergie linéaire délivrée.

Etape S9

Ce test est identique au test de l'étape S4 précitée.

Si un tir laser est en cours, les moyens électroniques de traitement 2 rebouclent sur l'étape S5 (acquisition d'une nouvelle image I(t+1) , ... )

Si aucun tir n'est effectué par l'opérateur Op (fin d'une séquence du traitement), les moyens électroniques de traitement 2 affichent sur l'écran 20 la dernière image acquise I(t) de la zone traitée avec la cartographie des doses d'énergie linéaire $E_{ij}$.

A titre d'exemple, on a représenté sur la figure 5b, un exemple de cartographie des doses d'énergie délivrées en début de traitement, et sur la figure 5c un exemple de cartographie des doses d'énergie délivrées en fin de traitement.

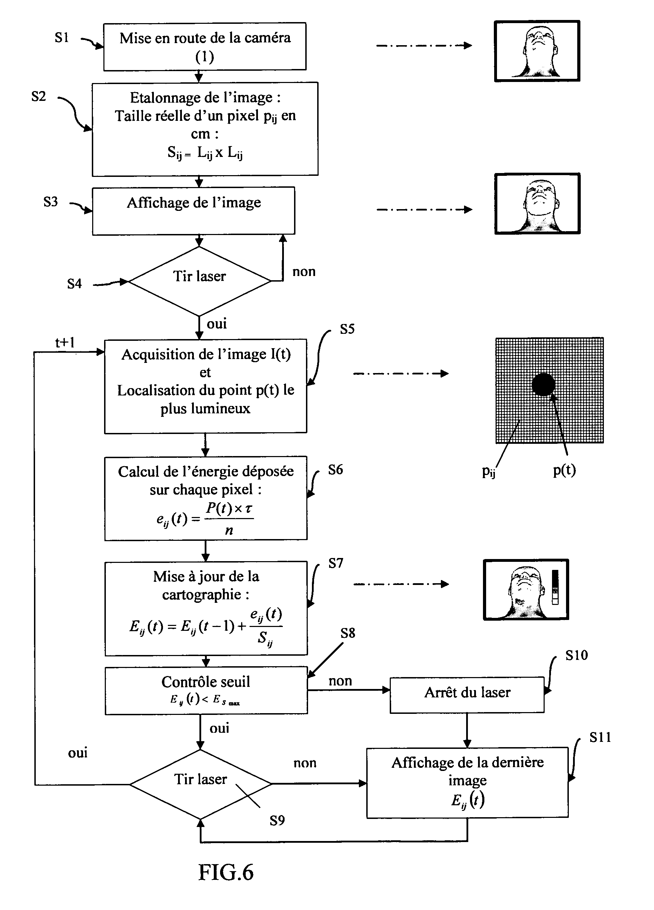

Exemple d'algorithme de cartographie pour un traitement de type lipolyse ou de type remodelage ou cicatrisation de la peau - Figure 6

Cet algorithme permet de cartographier les doses d'énergie surfaciques délivrées au cours d'un traitement de type lipolyse, au cours duquel l'extrémité distale de la fibre optique F est introduite dans l'hypoderme, et est déplacée de manière usuelle dans un plan par l'opérateur (Op) en sorte de balayer une surface à traiter. Cet algorithme convient également pour tout traitement par irradiation électromagnétique au cours duquel l'extrémité distale de la fibre optique est introduite et déplacée dans le plan sous-dermique SD (par exemple traitement de remodelage ou de cicatrisation de la peau décrit ci-après) ou au cours duquel l'extrémité distale de la fibre optique est introduite et déplacée dans le derme.

Cet algorithme se différencie de l'algorithme précité de la figure 5 pour le traitement endoveineux uniquement par le calcul de l'étape S7, qui prend en compte la surface $S_{ij}$ d'un pixel (et non plus la largeur $L_{ij}$ d'un pixel), la valeur d'énergie $E_{ij}(t)$ calculée étant une énergie surfacique (énergie par unité de surface).

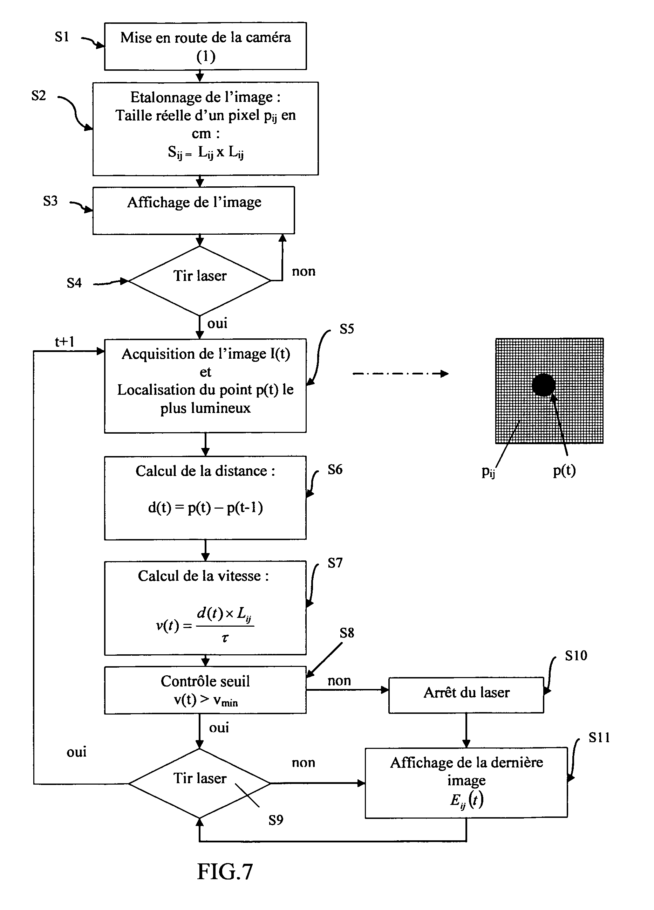

Exemple d'algorithme de contrôle de la vitesse de déplacement du spot d'irradiation - Figure 7

Dans cet algorithme, les étapes S1 à S3 de calibrage automatique de la caméra 1 préalables à la mise en oeuvre du traitement, ainsi que les étapes S4, S5 et S9 à S11 sont identiques respectivement aux étapes S1 à S5 et aux étapes S9 à S11 de l'algorithme de la figure 5, et ne seront donc pas plus amplement décrites.

Cet algorithme de la figure 7 permet (étapes S5 à S7) de calculer automatiquement et en temps réel la vitesse linéaire de déplacement v(t) du spot d'irradiation (S) au cours d'un traitement (quel que soit le type de traitement sous-cutané ou intra-cutané), et de contrôler automatiquement (étapes S8) si cette vitesse v(t) est supérieure à un seuil de vitesse minimum ($v_{min}$) prédéfini, et de préférence réglable en fonction du traitement. Si la vitesse calculée v(t) descend en dessous de ce seuil $v_{min}$ (ce qui correspond à une vitesse de retrait de la fibre optique par l'opérateur qui est trop faible et qui conduirait à délivrer des doses d'énergie électromagnétique trop importantes), la source 3 est arrêtée automatiquement par les moyens électroniques de traitement 2 (Etapes S10).

Le calcul de la vitesse v(t) effectué à l'étape S7 est obtenu à partir de deux positions [points lumineux p(t-1) et p(t)] du spot d'irradiation (S) dans deux images successives I(t-1) et I(t), et du calcul de la distance d(t) (Etape S6) entre ces deux positions, cette distance d(t) étant exprimée à l'étape S6 en nombre de pixels de l'image.

Dans une autre variante de réalisation, l'algorithme de traitement peut être modifié pour être plus complet et mettre en oeuvre pour un même traitement d'irradiation par rayonnement électromagnétique à la fois une cartographie des énergies délivrées ($e_{ij}(t)$ ou $E_{ij}(t)$) et un contrôle de la vitesse de déplacement v(t) du spot d'irradiation à l'extrémité distale de la fibre optique (fusion des algorithmes de la figure 5 ou de la figure 6 avec celui de la figure 7)

La solution de contrôle d'un traitement par irradiation sous-cutanée ou intra-cutanée au moyen d'un rayonnement électromagnétique de l'invention est particulièrement (mais pas exclusivement) adaptée pour contrôler le nouveau procédé de remodelage ou de cicatrisation de la peau de l'invention, dont une variante de réalisation va à présent être décrite.

Les principales étapes de ce procédé de remodelage ou cicatrisation de la peau sont les suivantes.

En référence à la figure 4, l'opérateur Op introduit dans le plan sous-dermique SD, l'extrémité distale de la fibre optique F et déplace selon un mouvement continu ou discontinu l'extrémité distale de la fibre optique F dans ce plan sous dermique SD en tirant sur la fibre optique F. Pendant ce déplacement de la fibre optique, l'opérateur OP commande la source laser 3 pour délivrer (de manière continue ou discontinue) des doses d'énergie électromagnétique dans ce plan sous-dermique SD à différentes positions de l'extrémité distale de la fibre optique.

La puissance P(t) de la source 3 ou la vitesse v(t) linéaire de déplacement du spot d'irrdation S dans le plan sous dermique SD sont contrôlées de telle sorte que température dans le plan sous-dermique SD soit comprise entre environ entre 45°C et 55 °C, et plus préférentiellement encore entre 48°C et 52°C. Une température supérieure ou égale à 45° est suffisante pour obtenir un remodelage efficace de la peau (diminution de la profondeur des rides, remodelage des zones de l'épiderme ayant un aspect « peau d'orange ») ou une meilleure cicatrisation de la peau, par un chauffage du collagène dans le derme et/ou par un chauffage des fibroblastes permettant la stimulation de la production de collagène dans le derme. Une température inférieure à 55°C permet d'éviter d'occasionner des dommages thermiques irréversibles dans le derme B ou dans l'épiderme A.

De préférence, la puissance P(t) de la source 3 est faible et inférieure ou égale à 5W et la vitesse de déplacement v(t) est comprise entre 20mm/s et 50mm/s.

Le choix de la vitesse de déplacement v(t) pour une puissance donnée P(t) dépend de la longueur d'onde du spot d'irradiation (S).

A titre d'exemple, le tableau I ci-dessous fournit des valeurs optimales de couple (P(t) /V(t)) pour différentes longueurs d'onde.

Tableau I

| Longueur d'onde (nm) | 600 | 800 | 980 | 1100 | 1200 | 1400 |
|---|---|---|---|---|---|---|
| Puissance P(t) (W) | 5 | 5 | 5 | 5 | 5 | 5 |
| Vitesse v(t) (mm/s) | 50 | 20 | 10 | 15 | 20 | 60 |
| K (constante) = P(t)/v(t) | 0.1 | 0.25 | 0.5 | 0.33 | 0.25 | 0.08 |

Dans la plage de longueurs d'onde entre 800nm et 1200 nm, la vitesse v(t) minimale qu'il convient d'appliquer est suffisamment lente, pour être facilement mise en oeuvre manuellement par un opérateur Op. Par contre en dehors de cette plage, la vitesse minimale qu'il convient d'appliquer est relativement importante, ce qui rend le traitement plus risqué, car le moindre ralentissement peut entraîner une surchauffe très rapide des tissus. Plus généralement, la plage de longueurs d'onde 800nm- 1320nm est préférable pour la mise en oeuvre du procédé. Le procédé peut néanmoins être mis en oeuvre avec des longueurs d'onde situées en dehors de cette plage. Plus particulièrement dans un exemple particulier de réalisation, on met en oeuvre le procédé de telle sorte que la puissance P(t) de la source de rayonnement électromagnétique et la vitesse linéaire v(t) de déplacement du spot d'irradiation S dans le plan sous-dermique SD respectent la condition: P(t) = k.v(t), k étant une constante prédéfinie.

Plus particulièrement, on choisira k compris entre 0,1 et 0,5, pour une puissance exprimée en Watt et une vitesse exprimée en mm/s.

## Revendications

1. Procédé de contrôle d'un traitement au cours duquel on effectue une irradiation sous-cutanée ou intra-cutanée au moyen d'un rayonnement électromagnétique de traitement et éventuellement au moyen d'un rayonnement électromagnétique de visée, **caractérisé en ce qu'**on met en oeuvre les étapes suivantes :

   - acquisition de plusieurs images I(t) successives de la zone traitée au moyen d'un capteur (1) externe qui est sensible à la longueur d'onde ou dans la gamme de longueurs d'onde du rayonnement électromagnétique de traitement ou du rayonnement électromagnétique de visée, l'intervalle de temps ($\tau$) entre deux images successives [I(t-1) ; I(t)] étant connu,
   - détection et localisation dans chaque image I(t) d'un point lumineux p(t) correspondant au spot d'irradiation (S) du rayonnement électromagnétique de traitement ou du rayonnement électromagnétique de visée,
   - calcul pour chaque point lumineux p(t) de l'un au moins des paramètres suivants : l'énergie délivrée [$e_{ij}$(t) ou $E_{ij}$(t) ] à partir de la puissance P(t) du rayonnement électromagnétique de traitement et de l'intervalle de temps ($\tau$) entre deux images successives [I(t-1) ; I(t)] ; la vitesse de déplacement v(t) du spot d'irradiation (S) à partir des positions de deux points lumineux [p(t-1) ; p(t)] dans deux images différentes [I(t-1) ; I(t)] et de l'intervalle de temps entre ces deux images [I(t-1) ; I(t)].

2. Procédé selon la revendication 1, **caractérisé en ce que** le rayonnement électromagnétique détecté par le capteur (1) est le rayonnement électromagnétique de traitement.

3. Procédé selon la revendication 1 ou 2 , **caractérisé en ce que** le capteur (1) est une caméra CCD.

4. Procédé selon l'une des revendications 1 à 3, **caractérisé en ce que** la longueur d'onde du rayonnement électromagnétique détecté par le capteur (1) est comprise entre 600nm et 1400nm.

5. Procédé selon l'une des revendications 1 à 4, **caractérisé en ce qu'**on réalise une cartographie des doses d'énergie délivrées au cours du traitement en associant, à chaque point lumineux détecté p(t), l'énergie délivrée [$e_{ij}$(t) ou $E_{ij}$(t)] calculée pour ledit point lumineux p(t).

6. Procédé selon la revendication 5, **caractérisé en ce qu'**on affiche sur un écran (20) la cartographie des doses d'énergie délivrées au fur et à mesure du traitement.

7. Procédé selon la revendication 6, **caractérisé en ce que** la cartographie des doses d'énergie délivrées est affichée en superposition avec une image réelle de la zone traitée acquise au moyen du capteur (3).

8. Procédé selon l'une des revendications 1 à 7, **caractérisé en ce qu'**on calcule, pour chaque point lumineux p(t), l'énergie linéaire ou surfacique $E_{ij}$(t), en prenant en compte la largeur réelle ($L_{ij}$) ou surface réelle ($S_{ij}$) d'un pixel d'une image I(t).

9. Procédé selon l'une des revendications 1 à 8, **caractérisé en ce qu'**on compare chaque valeur calculée pour le paramètre d'énergie [$e_{ij}$(t) ou $E_{ij}$(t)] avec au moins un seuil maximum prédéfini, et on arrête automatiquement le rayonnement électromagnétique de traitement lorsque la valeur d'énergie calculée est supérieure à ce seuil.

10. Procédé selon l'une des revendications 1 à 9, **caractérisé en ce qu'**on compare chaque valeur calculée pour le paramètre de vitesse v(t) avec au moins un seuil minimum prédéfini ($V_{min}$), et on arrête automatiquement le rayonnement électromagnétique de traitement lorsque la valeur de vitesse v(t) calculée est inférieure à ce seuil.

11. Système de contrôle d'un traitement au cours duquel on effectue une irradiation sous-cutanée ou intra-cutanée au moyen d'un rayonnement électromagnétique de traitement et éventuellement au moyen d'un rayonnement électromagnétique de visée , **caractérisé en ce qu'**il comprend un capteur (1) qui est sensible à la longueur d'onde ou dans la gamme de longueurs d'onde du rayonnement électromagnétique de traitement ou du rayonnement électromagnétique de visée, et qui permet l'acquisition de plusieurs images I(t) successives de la zone traitée, avec un intervalle de temps ($\tau$) connu entre deux images successives [I(t-1) ; I(t)], et des moyens de traitement (2), qui sont conçus pour traiter automatiquement les images I(t) acquises par le capteur, de manière à détecter et localiser dans chaque image I(t) le spot d'irradiation (S) du rayonnement électromagnétique de traitement ou du rayonnement électromagnétique de visée sous la forme d'un point lumineux p(t), et à calculer pour chaque point lumineux p(t) l'un au moins des paramètres suivants : l'énergie délivrée [$e_{ij}$(t) ou $E_{ij}$(t) ] à partir de la puissance P(t) du rayonnement électromagnétique de traitement et de l'intervalle de temps ($\tau$) entre deux images successives [I(t-1) ; I(t)] ; la vitesse de déplacement v(t) du spot d'irradiation (S) à partir des positions de deux points lumineux [p(t-1) ; p(t)] dans deux images différentes [I(t-

1) ; I(t)] et de l'intervalle de temps entre ces deux images [I(t-1) ; I(t)].

12. Système selon la revendication 11, **caractérisé en ce que** le capteur

(1) est une caméra CCD.

13. Système selon l'une des revendications 11 ou 12, **caractérisé en ce que** les moyens de traitement (2) sont conçus pour calculer une cartographie des doses d'énergie délivrées au cours du traitement à partir de chaque point lumineux détecté p(t) et de l'énergie délivrée [$e_{ij}$(t) ou $E_{ij}$(t)] calculée pour ledit point lumineux p(t).

14. Système selon la revendication 13, **caractérisé en ce que** les moyens de traitement (2) comportent un écran (20) et sont conçus pour afficher sur cet écran la cartographie des doses d'énergie au fur et à mesure du traitement.

15. Système selon la revendication 14, **caractérisé en ce que** les moyens de traitement (2) sont conçus pour afficher sur ledit écran la cartographie des doses d'énergie en superposition avec une image réelle de la zone traitée acquise au moyen du capteur (1).

16. Système selon l'une des revendications 11 à 15, **caractérisé en ce** les moyens de traitement (2) sont conçus pour calculer, pour chaque point lumineux p(t), l'énergie linéaire ou surfacique $E_{ij}$(t), en prenant en compte la largeur réelle ($L_{ij}$) ou surface réelle ($S_{ij}$) d'un pixel d'une image I(t).

**17.** Système selon l'une des revendications 11 à 16, **caractérisé en ce que** les moyens de traitement (2) sont conçus pour comparer chaque valeur calculée pour le paramètre d'énergie [$e_{ij}(t)$ ou $E_{ij}(t)$] avec au moins un seuil maximum prédéfini, et pour commander l'arrêt automatique du rayonnement électromagnétique de traitement lorsque la valeur d'énergie calculée est supérieure à ce seuil.

**18.** Système selon l'une des revendications 11 à 17, **caractérisé en ce que** les moyens de traitement (2) sont conçus pour comparer chaque valeur calculée pour le paramètre de vitesse $v(t)$ avec au moins un seuil minimum prédéfini ($v_{min}$) et pour commander l'arrêt automatique du rayonnement électromagnétique de traitement lorsque la valeur de vitesse calculée est inférieure à ce seuil.

**19.** Appareil médical permettant un traitement par irradiation sous-cutanée ou intra-cutanée au moyen d'un rayonnement électromagnétique de traitement, et comportant au moins une première source (3) permettant de délivrer un rayonnement électromagnétique de traitement, et éventuellement une deuxième source permettant de délivrer un rayonnement électromagnétique de visée, **caractérisé en ce qu'**il comprend en outre un système de contrôle visé à l'une des revendications 11 à 18.

**20.** Appareil médical selon la revendication 19, **caractérisé en ce que** la première source de rayonnement électromagnétique (3) communique avec les moyens de traitement (2) du système de contrôle pour fournir aux moyens de traitement la valeur de la puissance $P(t)$ de la source (3).

**21.** Appareil médical selon la revendication 19 ou 20, **caractérisé en ce que** les moyens de traitement (2) du système de contrôle sont aptes à commander l'arrêt de la première source (3) de rayonnement électromagnétique

**22.** Procédé de remodelage ou de cicatrisation de la peau par irradiation au moyen d'un rayonnement électromagnétique, **caractérisé en ce que** l'irradiation est réalisée dans le plan sous-dermique (SD).

**23.** Procédé selon la revendication 22, **caractérisé en ce qu'**on introduit dans le plan sous-dermique (SD) l'extrémité distale d'une fibre optique (F) reliée à une source (3) de rayonnement électromagnétique, **en ce qu'**on déplace l'extrémité distale de la fibre optique (F) dans ce plan sous-dermique (SD) en tirant sur la fibre optique, et on délivre au moyen de ladite source (3) de rayonnement électromagnétique des doses d'énergie électromagnétique dans ce plan sous-dermique (SD) à différentes positions de l'extrémité distale de la fibre optique (F).

**24.** Procédé selon la revendication 22 ou 23, **caractérisé en ce** la puissance $P(t)$ de la source (3) de rayonnement électromagnétique et la vitesse linéaire $v(t)$ de déplacement du spot d'irradiation (S) dans le plan sous-dermique (SD) respectent la condition : $P(t) = k.v(t)$, k étant une constante prédéfinie.

**25.** Procédé selon la revendication 24, **caractérisé en ce que** k est compris entre 0,1 et 0,5, pour une puissance $P(t)$ exprimée en Watt et une vitesse $v(t)$ exprimée en mm/s.

**26.** Procédé selon l'une des revendications 22 à 25, **caractérisé en ce que** la longueur d'onde du rayonnement électromagnétique est comprise entre 800 nm et 1320nm.

**27.** Procédé selon l'une des revendications 22 à 26, **caractérisé en ce que** la puissance de la source de rayonnement électromagnétique est inférieure ou égale à 5W.

**28.** Procédé selon l'une des revendications 22 à 27, **caractérisé en ce que** la vitesse linéaire $v(t)$ de déplacement de l'extrémité distale de la fibre optique est inférieure ou égale à 50mm/s, et de préférence comprise entre 20mm/s et 50mm/s.

**29.** Utilisation du procédé de contrôle visé à l'une des revendications 1 à 10 ou du système de contrôle visé à l'une des revendications 11 à 18, pour contrôler un traitement de lipolyse ou un traitement endoveineux.

**30.** Utilisation du procédé de contrôle visé à l'une des revendication 1 à 10 ou du système de contrôle visé à l'une des revendications 11 à 18, pour contrôler un procédé de remodelage ou de cicatrisation de l'épiderme visé à l'une des revendications 22 à 28.

Fig.1

Fig.2

Fig.3

Fig.4

S1 — Mise en route de la caméra (1)

S2 — Etalonnage de l'image : Taille réelle d'un pixel $p_{ij}$ en cm : $S_{ij} = L_{ij} \times L_{ij}$

S3 — Affichage de l'image

S4 — Tir laser — non

oui

t+1

S5 — Acquisition de l'image I(t) et Localisation du point p(t) le plus lumineux

$p_{ij}$    p(t)

S6 — Calcul de l'énergie déposée sur chaque pixel : $e_{ij}(t) = \dfrac{P(t) \times \tau}{n}$

S7 — Mise à jour de la cartographie : $E_{ij}(t) = E_{ij}(t-1) + \dfrac{e_{ij}(t)}{L_{ij}}$

S8 — Contrôle seuil $E_{ij}(t) < E_{L\,max}$ — non — S10 — Arrêt du laser

oui

oui

Tir laser — non — S11 — Affichage de la dernière image $E_{ij}(t)$

S9

FIG.5

Fig.5a

Fig.5b

Fig.5c

S1 — Mise en route de la caméra
(1)

S2 — Etalonnage de l'image :
Taille réelle d'un pixel $p_{ij}$ en cm :
$S_{ij} = L_{ij} \times L_{ij}$

S3 — Affichage de l'image

S4 — Tir laser → non

oui

t+1 → Acquisition de l'image I(t)
et
Localisation du point p(t) le plus lumineux — S5

$p_{ij}$     p(t)

Calcul de l'énergie déposée sur chaque pixel : — S6
$$e_{ij}(t) = \frac{P(t) \times \tau}{n}$$

Mise à jour de la cartographie : — S7
$$E_{ij}(t) = E_{ij}(t-1) + \frac{e_{ij}(t)}{S_{ij}}$$

S8 — Contrôle seuil
$E_{ij}(t) < E_{s\,max}$ → non → Arrêt du laser — S10

oui

oui — Tir laser → non → Affichage de la dernière image $E_{ij}(t)$ — S11

S9

FIG.6

S1 → Mise en route de la caméra
(1)

S2 → Etalonnage de l'image :
Taille réelle d'un pixel $p_{ij}$ en cm :
$S_{ij} = L_{ij} \times L_{ij}$

S3 → Affichage de l'image

S4 → Tir laser — non

oui

t+1 → Acquisition de l'image I(t)
et
Localisation du point p(t) le plus lumineux
S5

$p_{ij}$    p(t)

Calcul de la distance :

$d(t) = p(t) - p(t\text{-}1)$
S6

Calcul de la vitesse :

$v(t) = \dfrac{d(t) \times L_{ij}}{\tau}$
S7

Contrôle seuil
$v(t) > v_{min}$
S8 — non → Arrêt du laser S10

oui

Tir laser — non → Affichage de la dernière image
$E_{ij}(t)$
S11

oui

S9

FIG.7

## RAPPORT PARTIEL DE RECHERCHE EUROPEENNE

**Office européen des brevets**

qui selon la règle 63 de la Convention sur le brevet européen est considéré, aux fins de la procédure ultérieure, comme le rapport de la recherche européenne

Numéro de la demande

EP 07 37 0014

### DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | US 2005/154380 A1 (DEBENEDICTIS LEONARD C [US] ET AL DEBENEDICTIS LEONARD C [US] ET AL) 14 juillet 2005 (2005-07-14) * alinéas [0012] - [0024], [0042], [0055], [0063] - [0066], [0073] - [0085]; figures 1,2,8 * ----- | 11-14, 16-21 | INV. A61B18/20 |
| X | US 2007/049996 A1 (BLACK JOHN F [US]) 1 mars 2007 (2007-03-01) * alinéas [0011] - [0029], [0037], [0042], [0047] - [0053], [0057] - [0059] * ----- | 11-17, 19,20 | |
| X | WO 00/62700 A (KONINKL PHILIPS ELECTRONICS NV [NL]) 26 octobre 2000 (2000-10-26) * page 14, ligne 30 - page 19, ligne 23 * ----- | 11,12, 19,20 | |
| X | US 2007/005047 A1 (FERREN BRAN [US] ET AL) 4 janvier 2007 (2007-01-04) * alinéas [0050], [0052], [0058], [0065] - [0068]; figures 5,12,18 * ----- | 11,12,19 | |

-/--

**DOMAINES TECHNIQUES RECHERCHES (IPC)**

A61B

### RECHERCHE INCOMPLETE

La division de la recherche estime que la présente demande de brevet, ou une ou plusieurs revendications, ne sont pas conformes aux dispositions de la CBE au point qu'une recherche significative sur l'état de la technique ne peut être effectuée, ou seulement partiellement, au regard de ces revendications.

Revendications ayant fait l'objet d'une recherche complète:

Revendications ayant fait l'objet d'une recherche incomplète:

Revendications n'ayant pas fait l'objet d'une recherche:

Raison pour la limitation de la recherche:

voir feuille supplémentaire C

| Lieu de la recherche | Date d'achèvement de la recherche | Examinateur |
|---|---|---|
| Munich | 9 janvier 2008 | Link, Tatiana |

**Office européen**
**des brevets**

**RAPPORT PARTIEL**
**DE RECHERCHE EUROPEENNE**

Numero de la demande

EP 07 37 0014

| | | DOCUMENTS CONSIDERES COMME PERTINENTS | | CLASSEMENT DE LA DEMANDE (IPC) |
|---|---|---|---|---|
| Catégorie | Citation du document avec indication, en cas de besoin, des parties pertinentes | | Revendication concernée | |
| X | WO 00/53261 A (ASAH MEDICO A S [DK]; ASAH BJARNE [DK]; BALLE PETERSEN OLAV [DK]; DOLL) 14 septembre 2000 (2000-09-14) * page 1, ligne 11 - page 3, ligne 30 * * page 7, ligne 8 - page 10, ligne 4 * * page 14, ligne 27 - page 18, ligne 27 * * page 30, ligne 28 - page 32, ligne 16 * ----- | | 11,12, 15,19 | |

DOMAINES TECHNIQUES
RECHERCHES (IPC)

EPO FORM 1503 03.82 (P04C11)

**Office européen**

**des brevets**

**RECHERCHE INCOMPLETE**

**FEUILLE SUPPLEMENTAIRE C**

Numéro de la demande

EP 07 37 0014

Revendications ayant fait l'objet de recherches complètes:
       11-21

Revendications n'ayant pas fait l'objet de recherches:
       1-10,22-30

Raison pour la limitation de la recherche (invention(s) non
brevetable(s)):

Article 53 (c) CBE - Méthode de traitement chirurgical du corps humain ou
animal

**ANNEXE AU RAPPORT DE RECHERCHE EUROPEENNE
RELATIF A LA DEMANDE DE BREVET EUROPEEN NO.**

EP 07 37 0014

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

09-01-2008

| Document brevet cité au rapport de recherche | | Date de publication | Membre(s) de la famille de brevet(s) | | Date de publication |
|---|---|---|---|---|---|
| US 2005154380 | A1 | 14-07-2005 | EP | 1701666 A1 | 20-09-2006 |
| | | | JP | 2007531558 T | 08-11-2007 |
| | | | KR | 20060129278 A | 15-12-2006 |
| | | | WO | 2005063138 A1 | 14-07-2005 |
| US 2007049996 | A1 | 01-03-2007 | AUCUN | | |
| WO 0062700 | A | 26-10-2000 | AT | 306861 T | 15-11-2005 |
| | | | DE | 60023236 D1 | 24-11-2005 |
| | | | DE | 60023236 T2 | 11-01-2007 |
| | | | ES | 2248075 T3 | 16-03-2006 |
| | | | JP | 2002541906 T | 10-12-2002 |
| | | | US | 7108690 B1 | 19-09-2006 |
| US 2007005047 | A1 | 04-01-2007 | AUCUN | | |
| WO 0053261 | A | 14-09-2000 | AU | 3147200 A | 28-09-2000 |
| | | | US | 6676654 B1 | 13-01-2004 |
| | | | US | RE38670 E1 | 14-12-2004 |

Pour tout renseignement concernant cette annexe : voir Journal Officiel de l'Office européen des brevets, No.12/82

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 6206873 B
- US 5954710 A
- US 20060224148 A
- US 4564011 A
- US 5531739 A
- US 6398777 B
- US 20040199151 A
- WO 2006107522 A
- US 6659999 B
- US 20030040739 A
- US 5370642 A

**Littérature non-brevet citée dans la description**

- **A. CAPON et al.** Laser Assisted Skin Closure (LASC) by using a 815- nm Diode-Laser System Accelerates and Improves Wound healing. *Lasers in Surgery and Medicine,* 2001, vol. 28, 168-175